# EUROPEAN PATENT SPECIFICATION

(11) **EP 0 760 103 B1**
(45) Date of publication and mention of the grant of the patent: **20.10.1999**
(21) Application number: 95916861.8
(22) Date of filing: 09.05.1995
(51) Int. Cl.: G01N 33/80, G01N 33/538, G01N 33/541

(54) **SOLID-PHASE FILTRATION METHOD FOR ANTIGEN AND ANTIBODY ASSAYS IN BLOODGROUP SEROLOGY, AND TEST KIT**
FESTPHASEN-FILTRATIONSVERFAHREN ZUR ANTIGEN- UND ANTIKÖRPERPRÜFUNG IN DER BLUTGRUPPEN-SEROLOGIE, UND TESTSATZ
PROCEDE DE FILTRATION EN PHASE SOLIDE POUR DOSAGES D'ANTIGENES ET D'ANTICORPS EN SEROLOGIE DE GROUPES SANGUINS, ET NECESSAIRE D'ESSAI

(30) Priority: 10.05.1994 NL 9400777
(43) Date of publication of application: 05.03.1997
(73) Proprietor: Stichting Centraal Laboratorium van de Bloedtransfusiedienst van het Nederlandse Rode Kruis, 1066 CX Amsterdam (NL)
(72) Inventor: DEN BOER, Pieter, Johannes, NL-2313 KH Leiden (NL); VAN DER DONK, Eric, Marinus, Maria, NL-3435 ZE Nieuwegein (NL); VAN EIJK, Ronald, Victor, Wilhelmus, NL-3981 GM Bunnik (NL)
(74) Representative: Smulders, Theodorus A.H.J., Ir.
(86) International application number: NL9500165
(87) International publication number: WO9530904

(56) References cited:
- EP-A- 0 542 655
- EP-A- 0 594 506
- FR-A- 2 660 437
- FR-A- 2 673 472
- GB-A- 2 250 342

## Description

### Background of the invention

The purpose of bloodgroup assays and antibody identification in clinical work is often to obtain compatible erythrocyte preparations for transfusion. The invention concerns the detection and identification of antibodies and antigens and can be used for bloodgroup assay, antibody screening and identification, and performing cross tests. The test is not based on hemagglutination but on the solid-phase principle, with elimination of washing steps.

In a bloodgroup, specific antigenic determinants on the cell membrane are involved. The information for expressing bloodgroup antigens is fixed on a gene level and hereditary. Bloodgroup antigens can give rise to the formation of antibodies. Specific antibodies to bloodgroup antigens are mostly formed after immunization with the corresponding antigen. An exception to this is formed by the so-called "naturally occurring" antibodies which without apparent immunization are demonstrable in the serum (anti-A and anti-B). The presence of antibodies plays an important role in blood transfusion, pregnancy and auto-immunity.

Antibodies can be classified in different ways:
1. xeno, allo and auto-antibodies;
2. regular and irregular antibodies;
3. naturally occurring and acquired antibodies;
4. complete (IgM) and incomplete (IgG) antibodies.

Blood transfusion reactions which are induced by alloantibodies against erythrocytes are called hemolytic transfusion reactions because they are mostly accompanied by an often very strongly accelerated breakdown of erythrocytes. The point is therefore to prevent hemolytic transfusion reactions by careful bloodgroup examination.

Bloodgroup antibodies are nearly always immunoglobulins of the IgG type or the IgM type. The antigen-antibody interaction is dependent *inter alia* on ionic bonding, hydrogen bridges and hydrophobic effects (displacement of water). The strength of a bond between a binding site of an antibody and an epitope is designated by affinity. Antibodies which are able to agglutinate erythrocytes under all conditions are called agglutinins or complete antibodies (IgM). Antibodies which do bind to erythrocytes but give no agglutination (sensitization) are called incomplete antibodies (mostly IgG).

The detection of erythrocyte antigens and corresponding antibodies often takes place by means of agglutination reactions. Agglutination reactions can be allowed to take place in a physiological salt solution. In practice, this is not always optimal. A number of tests can be rendered more sensitive by using a number of aids, such as the use of medium having a low ionic strength (low ionic medium), proteolytic enzymes (e.g. bromelin, papain or ficin), polycations (e.g. polybrene), macromolecules (e.g. albumin), or polymers (e.g. polyethylene glycol, PEG).

An important and much used test is the antiglobulin or Coombs test. The antiglobulin test is based on the principle that erythrocytes loaded with, for instance, antibodies of the IgG type can be agglutinated by antiglobulin serum. This is the most important test for demonstrating incomplete antibodies. The antiglobulin test was described by Moreschi in 1908 (Zbl. Bakt 46: 49) and reintroduced in 1945 by Coombs et al. (Lancet 2: 15, Brit. J. Exp. Path. 26: 255).

The antiglobulin test can be distinguished into three phases. The first phase is the sensitization phase. During this phase, antibodies bind to the corresponding antigen structures on the erythrocytes. If sufficient binding has taken place, the second phase takes place, viz. the washing phase. In this phase, all non-bound antibodies are removed from the incubation mixture. Insufficient removal of non-bound antibodies can lead to inactivation of the antiglobulin serum in that these antibodies bind to the antiglobulin. The third phase is the antiglobulin phase. In it, antiglobulin is added to the washed sensitized cells, so that the sensitized cells are coupled to each other.

There is a large variety of bloodgroup assay methods. The most important techniques at present are the tube method, the gel test and tests in microtiter plates. A distinction can be made between techniques which are based on hemagglutination and techniques which are based on the solid-phase principle.

### a. Tests based on agglutination:

The tube test is a much used test which also allows prolonged incubations. The erythrocytes can be allowed to settle or be centrifuged to accelerate the agglutination reaction. Evaluation of the antigen-antibody reaction occurs by gently tapping the tube and then rotating the tube (tip and roll). A disadvantage of this method is that it is difficult to automate. The Coombs test requires very thorough and frequent washing. In addition, the reading of the test must be done immediately by an experienced person and the result of the test cannot be preserved. Because the reading of the test occurs manually, its reproducibility is not optimal either. In addition, automation of this assay method is also difficult to realize.

Another test based on agglutination is described in EP-A-0,542,655 and FR-2,673,472 (Institut Jacques Boy). The test described in these applications utilizes a filter principle. After incubation of erythrocytes and (anti)serum, agglutinates which have formed are retained on a combination of inert filter materials, including a layer of glass beads. For the transport of the erythrocytes which may or may not be agglutinated, use is made of an elution step (through-suction of the medium using a moisture-absorbing material). This method can be used directly for performing tests involving agglutinating antibodies. However, if non-agglutinating antibodies (for instance IgG antibodies in the Coombs test) are involved, erythrocytes will first have to be washed after the incubation period in order to remove the non-bound antibodies. Only then can these erythrocytes (sensitized or not) be introduced into the test system in the presence of antiglobulin serum, whereafter agglutination can take place. So the disadvantage of this test system is that in the antiglobulin test the incubation phase and the washing phase cannot take place in the same reaction vessel.

As indicated, the washing step in the Coombs test takes up much time. Graham et al. (Transfusion 1982, 22: 408; P.L. Mollison, Blood transfusion in clinical medicine, Blackwell, Oxford, 1983, p. 512) developed a new principle which rendered the washing step redundant. This principle was used by Ortho Diagnostic Systems Inc. in a test system (Simwash). According to this system, the erythrocytes are separated from the serum by means of a centrifugation step. The separation is based on the fact that the specific gravity of serum (1.03) is lower than that of erythrocytes (1.09). Now, if a mixture of cells and serum is applied on top of a layer of medium of a density between the density of the cells and that of the serum, the erythrocytes will be separated by the centrifugation step from the serum (containing the non-bound antibodies). Thus the (heavier) erythrocytes are centrifuged from the original incubation mixture. In this way a triple or quadruple washing step is reduced to a single centrifugation step. Then the sensitized cells can be incubated with antiglobulin serum. However, the system proved not to be sensitive enough. A further simplification has been carried through by centrifuging the erythrocytes through a Sephadex gel or through glass beads. The use of transparent, inert, solid particles for separating agglutinates and non-agglutinates was described by Dalton et al. in 1970 (Becton Dickinson & Co., US Patent 3,492,396).

A system where use is made of gel material for the detection of erythrocyte-antibody reactions has been described by LaPierre et al. (Transfusion 30: 109-113, 1990; and EP-A-0,194,212 and EP-A-0,305,337 and US Patent 5,338,689). This system combines the principles of Simwash and the use of solid particles for separating agglutinates and non-agglutinates and the detection of agglutinates. In the test, use is made of small columns filled with Sephadex gel. For the detection of agglutinins use is made of a neutral gel, and for the antiglobulin test use is made of a gel which contains antiglobulin serum. As a third possibility, a gel may also contain specific antibodies to erythrocyte-antigens. After an incubation the gel columns are centrifuged. In the case of a negative reaction, all erythrocytes end up at the bottom of the tube; if the test is positive, the erythrocytes will be captured on top of or in the gel and in the case of weak reactions, erythrocytes will settle partly.

A major advantage of this test system is that the antiglobulin test requires no separate washing steps (as is the case, for instance, with the tube test and the test described in EP-A-0,542,655 ). In fact, during the centrifugation a separation of erythrocytes and serum or plasma constituents takes place. A second advantage is the fact that the results of the test can be properly fixed. The test described is used by DiaMed AG (DiaMed-ID Microtyping System) and Diagast Laboratoires (Chromatest). A comparable system, Ortho Biovue System (Ortho Diagnostic Systems), utilizes glass beads instead of gel material. A disadvantage of these tests is that a special centrifuge is required for the correct performance of the test. Automatic reading of the test also requires special reading equipment. Moreover, mixed patterns may arise, with a part of the erythrocytes ending up at the bottom of the tube.

### b. Tests based on the solid-phase principle:

Another approach is the use of the solid-phase principle as an alternative to agglutination reactions for bloodgroup assay, antibody screening and identification, and cross testing. The use and advantages of the use of solid-phase techniques in the areas mentioned have been described by Rosenfield (Abstracts, 15th Cong. Int. Soc. Blood Trans., Paris pp. 27-33, 1976; US Patent 4,275,053, 1981). Here, *inter alia* erythrocytes were used which were coupled to the surface of plastic tubes. More recently, systems have been described by Plapp et al. (Am. J. Clin. Path. 82: 719-721, 1984), Bayer et al. (US Patent 4,608,246, 1986), Rachel et al. (Transfusion 25: 24-26, 1985), Plapp et al. (The Lancet 1465-1466, 1986) and Uthemann et al. (US Patent 4,925,786, Transfusion 30: 114-116, 1990).

Microtiter plates with the solid-phase principle are employed by, among others, Biotest AG (Solidscreen II for antibody diagnostics), Immucor Inc. (Immunocapture Capture-R systems for antibody screening and identification) and CLB (Microtype for typing erythrocyte antigens). The Capture-R system utilizes a solid phase to which membranes of erythrocytes are bound. During the incubation with serum or plasma from donors or patients, erythrocyte-specific antibodies can bind to these membranes. The washing phase, in which the excess non-bound antibodies are removed, is followed by the addition of indicator erythrocytes to which anti-IgG is bound. If in the plasma or serum specific antibodies were present, bridge formation will arise between the antibody bound to the solid phase and the indicator erythrocytes. Accordingly, this system concerns a modification of the antiglobulin test. Solidscreen (antibody screening and identification) and Microtype (antigen typing) utilize a solid phase to which human IgG is bound. In the wells the incubation of erythrocytes and plasma or serum takes place. If corresponding antibodies are present, sensitization of erythrocytes takes place. The sensitized cells are then washed to remove the non-bound antibodies. Detection takes place by adding antiglobulin serum, so that the sensitized cells are bound to the IgG on the solid phase. In this kind of systems it is usual for positive reactions to be characterized by a monolayer of erythrocytes on the solid phase. In the case of negative results, no monolayer is formed.

A disadvantage of the known tests is that they require frequent washing to remove the excess non-bound antibodies. Incomplete washing away of antibodies may lead to inactivation of the antiglobulin. An advantage is that small amounts of sample will suffice, that for carrying out the test, use can be made of automatic pipetting devices which are suitable for microtiter plates, and that automatic processing of results is possible.

Recently, a test principle has been described which also utilizes the solid-phase principle but employs particulate material rather than a microtiter plate as solid phase (EP-A-0,594,506; Pasteur Sanofi Diagnostics). This publication describes a columnar test system. In the upper part of the column, the formation of an immune complex takes place by means of incubation in an aqueous medium. This formed immune complex is transported by means of centrifugation through a gel matrix on which a substance has been immobilized which is able to bind an antibody (or antigen) (porous affinity matrix). The reaction can be evaluated on the basis of the position of the immune complexes in the gel column. An important application of this test lies in the area of bloodgroup serology. In this case, the immune complexes consist of sensitized erythrocytes which are capable of binding to the affinity gel matrix. The test can be carried out in both tubes and in the columns of plastic cards as described by LaPierre (Transfusion 30: 109-113, 1990, and EP-A-0,194,212 and EP-A-0,305,337).

The disadvantage of this method is that carrying out the test requires special equipment. The centrifugation of the column cards should take place in special centrifuges. The cards must be positioned exactly radius-wise in the rotor. If reading is automatic, use has to be made of special reading equipment based on the "image analysis" principle.

### Detailed description of the invention

The invention relates to a method for demonstrating antibodies and antigens whose application lies mainly in the field of bloodgroup serology: bloodgroup assay, antibody screening and identification, and cross testing. In the invention a permeable solid phase (membrane) is used on which one or more immunoglobulin-binding substances are immobilized. According to the invention, sensitized erythrocytes can be bound to this permeable solid phase. Erythrocytes on which no antibodies (immunoglobulins) are present will not be bound to the solid phase and can simply pass it because the solid phase is permeable to erythrocytes. For carrying out the test, no separate wash phase is needed, as is the case with the current solid-phase systems based on the microtiter plate system. An advantage of the use of a membrane as a solid phase over a column gel matrix (as described in EP-A-0,594,506) is that carrying out the test does not require any special equipment, including special centrifuges, detection equipment and pipetting systems. The invention represents an improvement of the current solid-phase principle.

The invention accordingly provides a method for assaying in a sample an analyte consisting of a bloodgroup antigen present on erythrocytes or an antibody binding to such a bloodgroup antigen, comprising treating the sample with a reagent containing an antibody capable of binding to a bloodgroup antigen or containing a bloodgroup antigen present on erythrocytes, whereby a complex of bloodgroup antigen present on erythrocytes and antibody bound thereto is formed if the reagent contains an antibody binding to a bloodgroup antigen present in the sample or the sample contains an antibody binding to a bloodgroup antigen present in the reagent, further comprising separating this complex, if any has formed, from uncomplexed erythrocytes, and detecting the result, which method is characterized in that erythrocyte-containing complexes, if any have formed, are separated from uncomplexed erythrocytes by filtration through a solid phase which is permeable to the uncomplexed erythrocytes, on which phase an immunoglobulin-binding material is immobilized.

The invention further provides a test kit suitable for use in a method according to the invention for assaying a bloodgroup antigen present on erythrocytes or an antibody binding to such a bloodgroup antigen, comprising
(i) a reagent which contains an antibody capable of binding to a bloodgroup antigen or contains a bloodgroup antigen present on erythrocytes, and
(ii) a solid phase which is permeable to erythrocytes, on which an immunoglobulin-binding material is immobilized.

### Advantages of the invention

The invention described here has the following advantages over existing techniques:
1) the sensitivity of the test is high due to the use of immunoglobulin-binding substances having high affinities to the different immunoglobulins;
2) no wash step is needed for removing an excess of unbound immunoglobulins;
3) no use is made of the principle of agglutination;
4) the assay can be performed on existing equipment;
5) reading and interpreting the test can be simply carried out visually but can also be readily automated;
6) it is a user-friendly, uniform system; and
7) the obtained result can be preserved.

### Variants of the invention

The invention described can be applied to the current tests within bloodgroup serology, including screen tests for antibodies, identification of antibodies, serum reverse typing, cross-testing, antigen assay, bloodgroup assay and antibody titration. The invention can for instance be used for assaying the following bloodgroup antigens and antibodies to those antigens: AB0, Kell (K), cellano (k), Duffy (Fy^{a} and Fy^{b}), Kidd (Jk^{a} and Jk^{b}), Lutheran (Lu^{a} and Lu^{b}), the Rhesus system (D, E, e, C, c), MNS (S and s), Lewis (Le^{a} and Le^{b}), etc.

By way of example, the invention can be used for the following assays:
- Tests in which use is made of an antiserum of a known specificity and erythrocytes having an unknown antigen composition (antigen assay, AB0 forward bloodgrouping).
- Tests in which use is made of sera including an unknown antibody or unknown antibodies and erythrocytes having a known antigen composition (antibody screening, antibody identification, reversed bloodgrouping). In this connection, often use is made of a panel of erythrocytes with a known antigen composition. In addition, the test can also be used for the detection of auto-antibodies. In this case, on the erythrocytes antibodies are already bound to the corresponding antigens. So the erythrocytes are already sensitized and can bind directly to the permeable solid phase.
- Tests in which erythrocytes of unknown antigen composition and sera having therein antibodies of unknown specificities are used (cross test).

The tests performed in accordance with the present invention are all based on the same principle: erythrocytes to which antibodies are bound (sensitized erythrocytes) can bind to the permeable solid phase, while non-sensitized cells will pass the permeable solid phase.

The sample will typically consist of blood or a material derived therefrom, such as blood plasma, blood serum or a blood fraction, for instance erythrocytes isolated from blood. However, since the invention can also be used for other purposes, such as for the selection of hybridomas which produce monoclonal antibodies which bind to a bloodgroup antigen, the sample can also consist of materials of a different type, for instance of a supernatant of a hybridoma.

Essential is that the solid phase used is permeable. As suitable filter material for the solid phase, porous membranes can be used. In addition, the choice of immunoglobulin-binding substances is also of essential importance. Both points will be briefly explained here.

### a. Filter material

Porous membranes which can be used as filter material must satisfy a number of requirements. The membranes must have sufficiently large pores to allow non-sensitized erythrocytes to pass, the pore size will have to be greater than about 2 µm, preferably greater than about 3 µm. It is true that the erythrocytes have a diameter of 6-8 µm, but due to their deformability they can pass through pores which are smaller than their diameter. The pore size employed depends on the material used and the number of pores per unit area. Too small a number of pores will lead to clogging. At a relatively large pore size, the binding of sensitized erythrocytes will proceed inefficiently, so that not enough cells can be bound. A finer membrane structure will lead to a more intensive contact between the sensitized cells and the immunoglobulin-binding substance, enabling optimum binding of the cells. In order to enable an incubation, in combination with the solid phase a hydrophobic filter can be used, which prevents the incubation mixture from passing the solid phase too fast.

Another important property of the solid phase is the capacity to immobilize the immunoglobulin-binding substance. The bond must be so firm that bound erythrocytes remain coupled to the solid phase. Examples of filter material as solid phase are, for instance, cellulose-ester polymer, nitrocellulose, (modified) polyvinylidene fluoride (PVDF), (modified) polytetrafluoroethylene (PTFE), (modified) polyether sulfone, glass fiber, polycarbonate and modified nylon. Nitrocellulose is a much used material as membrane due to its high affinity to proteins and cellular macromolecules. Materials such as nitrocellulose and nylon can be modified so as to enable covalent binding to macromolecules. An important advantage of polycarbonate is the low non-specific binding of reagents (Penney et al., 1989; J. Immunol. Meth. 123: 185-192).

### b. Immunoglobulin-binding substances.

The choice of immunoglobulin-binding substances which can be immobilized on the solid phase is large. Useful are, for instance, polyclonal or monoclonal antibodies which are directed against immunoglobulin G (IgG), immunoglobulin A (IgA) and/or immunoglobulin M (IgM). In order to increase the sensitivity, or to detect a largest possible range of antibodies, it is also possible to immobilize a combination of several immunoglobulin-binding substances on the solid phase, or to use anti-Ig. Also, antibodies to the complement system can be used.

Another approach is to start from immunoglobulin-binding substances of a different origin, for instance from bacteria. Immunoglobulin-binding bacterial proteins have already drawn much attention and are used inter alia in immunology, biochemistry, and biotechnology. A number of Ig-binding proteins have been isolated and characterized.

An important exponent is the protein A, described in 1966, derived from *Staphylococcus aureus* (Forsgren et al. J.Immunol. 97: 822). This cell wall protein has a high affinity to *inter alia* human immunoglobulin G (subclasses IgG₁, IgG₂ and IgG₄).

In 1984 protein G, a cell wall protein originating from group C and G *Streptococcus,* was described (Björck et al. J. Immunol. 133: 969; Reis et al. J. Immunol. 132: 3091). This protein binds IgG antibodies of all four subclasses with a high affinity. This property renders it highly suitable for use in immunohematology and in particular for antibody and antigen assays in bloodgroup serology. Further, the affinity of protein G to immunoglobulins is greater than that of protein A, so that it is a forceful reagent for the detection of immunoglobulins. Protein G too is usable as an immunoglobulin-binding substance on membranes.

Recently, two bacterial proteins have been described which can bind immunoglobulins with a high affinity, viz. protein L and protein H.

Protein L occurs on the surface of some strains of the bacterial species *Peptostreptococcus magnus* (Infect. Immun. 58: 1217-1222; 1990). It is a protein capable of binding the K-light chain of immunoglobulins (J. Immunol. Methods 164: 33-40, 1993), both IgG, IgA and IgM bind with a high affinity (J. Biol. Chem. 264: 19740-19746; 1989). The protein can bind specifically to the variable part of the light chain without interfering with the antigen binding site.

Protein H is a new IgG-binding bacterial protein (*Streptococcus* spec.). This protein has a high affinity to the heavy chain and Fc fragments of IgG. There is no binding with IgM, IgA, IgD, or IgE (Akesson et al. Mol. Immunol. 27: 523-531; 1990).

But substances in which, by means of changes of genetic material, binding properties of different proteins have been brought together may be useful as well. Examples include protein L/G (Kihlberg et al. J. Biol Chem. 267: 25583; 1992) and protein A/G (ImmunoPure Immobilized Protein A/G, Pierce). In protein L/G the Ig-binding parts of protein L and protein G have been brought together in a molecule with which, in addition to a large number of intact human immunoglobulins, the light chain of immunoglobulins and Fc and Fab fragments can also be bound. In protein A/G the binding characteristics of proteins A and G are brought together in one molecule. Further, cell suspensions of the bacteria mentioned (Sigma), or IgG binding fragments of protein A (Sigma) can be suitable as immunoglobulin-binding substance.

A possible variant is the use of genetically modified bacteriophages (A.S. Kang et al., Proc. Natl. Acad. Sci. USA 88: 4363-4366). On the surface of these bacteriophages, functional Fab fragments have been incorporated. These Fab fragments can bind antibodies and sensitized erythrocytes. These bacteriophages can be immobilized on the permeable solid phase and can serve as immunoglobulin-binding substance.

In combination with the invention described, a centrifugation step can be employed, so that sensitized cells are separated from the serum or plasma. This step is comparable with the earlier indicated procedure which is used by Diamed AG, Ortho Diagnostic Systems, Diagast Laboratoires and Sanofi Pasteur. An advantage of this combination is that appreciably less immunoglobulin-binding substance needs to be present on the solid phase.

For the bloodgroup assay method where IgM antibodies are of importance (AB0 system), as solid phase a porous membrane can be used on which an IgM-binding substance is immobilized. As a result, the same system can also be used for the assay of other antigens where the corresponding antibody is of the IgM class. So in this system binding of IgM-sensitized erythrocytes takes place.

For antigen typing involving IgG antibodies, use can be made of a solid phase where an IgG-binding substance with a high affinity is used, which ensures optimum binding of sensitized cells to a solid phase. As immunoglobulin-binding substance, protein G can be used again. The affinity of protein G to IgG is higher than that of protein A, moreover protein G binds all four IgG subclasses, while protein A does not bind IgG₃. The advantage of this system is that it can also be used for antibody identification, antibody screening and cross-tests where IgG-antibodies are involved. A solid phase on which one or more immunoglobulin-binding substances are present which bind IgA, IgG, IgM and complement factors is most ideal since then the largest range of antibody specificities can be demonstrated.

According to another embodiment of the test, on the solid phase a monoclonal or polyclonal antibody is immobilized which is directed against an antigen present on erythrocytes. In this connection it is not of importance whether an IgG or an IgM antibody is involved because no agglutination needs to take place as in the tube or gel test. Erythrocytes which possess the antigen against which the antibody immobilized on the filter is directed, can bind to this solid phase. This method is highly suitable for bloodgroup assays for, for instance, the AB0 and Rhesus system.

The solid-phase principle can further be used for demonstrating IgM antibodies by using the earlier mentioned solid phase consisting of a membrane having, for instance, immobilized protein G thereon. In this case an anti-IgM antibody of the IgG class is coupled to the protein G. This test can for instance be used in "reverse typing" of the AB0 bloodgroup (in this assay the specificity of the naturally occurring allo-agglutinins in the serum or plasma of the patient or donor is examined). IgM alloantibodies from the serum/plasma of the patient or donor can bind to the anti-IgM antibody. Then the specificity of the bound IgM alloantibodies is determined by adding test erythrocytes. Test erythrocytes of, for instance, bloodgroup B will attach to the solid phase if the bound IgM alloantibody is of the anti-B specificity.

For carrying out the test it is preferred to use a test system of microtiter plate format (with the possibility of performing single or multiple tests) where the bottom has been replaced with the permeable solid phase in combination with a hydrophobic filter. The solid phase preferably comprises a filter on which protein G is immobilized. After an incubation phase in which cells and (anti)serum can enter into a bond, transport of cells which may or may not be sensitized can take place by means of a centrifugation step in which sensitized cells will attach to the solid phase and non-sensitized cells will pass the solid phase. In this case, carrying out the test may require the arrangement of a fluid-filled compartment under the solid phase. An advantage of this centrifugation step is that in this way the erythrocytes, which may or may not be sensitized, are separated from the incubation medium. Detection of the bound cells on the filter can simply be effected both visually and automatically.

### Examples

The invention will now be further explained in and by the following examples. However, it is of importance to see that these examples are given for illustrative purposes and illuminate the enablement of the invention but in no way constitute the definitive conditions of the different assay methods.

### Example 1

Nitrocellulose membrane filters (AE 99; Schleicher & Schuell, Dassel, Germany) with a pore size of 8 µm were used as filter material. The membranes (surface area 80 mm²) were coated with protein G (Pharmacia, Sweden) by means of incubation (18 hours at 4°C) in 400 µl PBS containing 500 µg protein G/ml. After the coating procedure the membranes were flushed in PBS and treated with blockbuffer (PBS with 1% bovine albumin) for 1 hour at room temperature. The membranes were placed in membrane containers (Swinnex SX, Millipore, Molsheim, France). Disposed under the membrane container was a cock. The membranes were flushed with a saline solution of low ionic strength: LISS (low ionic strength saline) consisting of 3 mM sodium phosphate, 61 mM sodium chloride and 240 mM glycine, pH 6.6.

Then 200 µl anti-D sensitized 0⁺ erythrocytes (3% suspension in PBS) was applied to the membrane. As long as the cock under the membrane container was closed, applied erythrocytes remained on the membrane, so that an incubation period could be realized. After a short incubation time (room temperature) elution with PBS took place. Elution was effected by opening the cock and adding buffer. The elution took place under the influence of gravity. The sensitized erythrocytes proved to be bound to the membrane. The erythrocytes could be detached from the membrane by elution with 0.1 M glycine/HCl, pH 3.0).

As control, the procedure was repeated with 0⁺ erythrocytes which were not sensitized. Here no binding of cells to the membrane filter was observed..

Identical results were obtained if LISS was used instead of PBS.

### Example 2

The experiment as described in Example 1 was carried out with respect to the Duffy bloodgroup system. 100 µl erythrocyte suspension (genotype Fy^{a+b-}) was incubated with 100 µl anti-Fy^{a} serum for 15 min at 37°C. After this sensitization phase the erythrocytes were washed in PBS and finally resuspended in 200 µl LISS. Of this suspension (3%), 100 µl was applied to the membrane. An incubation period of 15 min was followed by elution with 2 ml PBS. All erythrocytes proved to be bound to the membrane. The erythrocytes could be removed by elution with 1 ml 0.1 M glycine/HCl pH 3.0.

As control, the procedure was repeated with 0⁻ erythrocytes (phenotype Fy^{a-b+}). Here no binding of erythrocytes to the membrane was observed.

### Example 3

In this example the test is carried out in a microtiter plate. Nitrocellulose membrane filters (AE 99; Schleicher & Schuell, Dassel, Germany) with a pore size of 8 µm were used as solid phase filter material. The membranes (surface 30 mm²) were coated with protein G (Pharmacia, Sweden) by means of incubation (18 hours at 4°C) in 800 µl PBS containing 200 µg protein G per ml. After the coating procedure the membranes were flushed in PBS and treated with blockbuffer (PBS with 1% bovine albumin) for 2 hours at room temperature. The membranes were placed in Multiscreen microtiter plates (Millipore, Bedford, Massachusetts). In these microtiter plates the bottom has been replaced by a polycarbonate membrane having a pore size of 5 µm. This material is hydrophobic and under normal conditions does not allow the passage of supernatant fluid. As a result, applied erythrocytes remain on the membrane, so that an incubation period can be realized. Transport of erythrocytes can be effected by centrifugation or by means of elution by pressing the microtiter plate on filtering paper.

The membranes were flushed with PBS containing 1% dextran. Then 50 µl anti-D sensitized 0⁺ erythrocytes (3% suspension in PBS) was applied to the membrane. A short incubation time (room temperature) was followed by elution with PBS containing 1% dextran. The sensitized erythrocytes proved to be bound to the membrane. The erythrocytes could be detached from the membrane by elution with 0.1 M glycine/HCl, pH 3.0).

As control, the procedure was repeated with 0⁺ erythrocytes which were not sensitized. Here no binding of erythrocytes to the membrane was observed.

### Example 4

The experiment as described in Example 3 was carried out with nitrocellulose membrane filters, which, however, were not coated with protein G but with monoclonal IgM-antibodies, respectively anti-A, anti-B, anti-AB or anti-D.

Then 50 µl erythrocyte suspension (3% in PBS) was applied to the membrane, respectively 50 µl A-, B-, AB- and 0⁺ erythrocytes. A short incubation time (room temperature) was followed by elution with PBS containing 1% dextran. In the four cases described, all erythrocytes proved to be bound to the membrane. The erythrocytes could be detached from the membrane by elution with 0.1 M glycine/HCl, pH 3.0).
As control, the procedure was repeated with 0⁻ erythrocytes. In the four cases described, no binding of erythrocytes to the membrane was observed.

## Claims

1. A method for assaying in a sample an analyte consisting of a bloodgroup antigen present on erythrocytes or an antibody binding to such a bloodgroup antigen, comprising treating the sample with a reagent containing an antibody capable of binding to a bloodgroup antigen or containing a bloodgroup antigen present on erythrocytes, whereby a complex of blcodgroup antigen present on erythrocytes and antibody bound thereto is formed if the reagent contains an antibody binding to a bloodgroup antigen present in the sample or the sample contains an antibody binding to a bloodgroup antigen present in the reagent, further comprising separating this complex, if any has formed, from uncomplexed erythrocytes, and detecting the result, characterized in that erythrocyte-containing complexes, if any have formed, are separated from uncomplexed erythrocytes by filtration through a solid phase which is a porous membrane permeable to the uncomplexed erythrocytes, on which membrane an immunoglobulin-binding material is immobilized.

2. A method according to claim 1, characterized in that the sample consists of blood, blood plasma, blood serum, a blood fraction or a supernatant of a hybridoma.

3. A method according to claim 1, characterized in that a membrane of cellulose-ester polymer, nitrocellulose, (modified) polyvinylidene fluoride (PVDF), (modified) polytetrafluoroethylene (PTFE), (modified) polyether sulfone, glass fiber, (modified) polycarbonate, or (modified) polyamide (nylon which may or may not be modified) is used.

4. A method according to claim 1, characterized in that the porous membrane has pores of at least about 2 µm, preferably at least about 3 µm.

5. A method according to claim 1, characterized in that in combination with the porous membrane a hydrophobic filter is used.

6. A method according to claim 1, characterized in that as immunoglobulin-binding material an anti-Ig, anti-IgG, anti-IgA or anti-IgM is used.

7. A method according to claim 1, characterized in that as immunoglobulin-binding material an immunoglobulin-binding bacterial protein is used.

8. A method according to claim 7, characterized in that as immunoglobulin-binding bacterial protein, protein A, protein G, protein L or protein H is used.

9. A method according to claim 1, characterized in that as immunoglobulin-binding material a recombinant protein having immunoglobulin-binding properties, such as protein L/G or protein A/G, is used.

10. A method according to claim 1, characterized in that the filtration is promoted by centrifugation.

11. A method according to claim 1, characterized in that the filtration is preceded by a centrifugation in which the erythrocytes which may or may not be complexed are separated from the sample.

12. A method according to claim 1, characterized in that the filtration is promoted by centrifugation, while simultaneously separating from the sample the erythrocytes which may or may not be complexed.

13. A method according to claim 1, characterized in that the substance to be assayed is a bloodgroup antigen present on erythrocytes.

14. A method according to claim 1, characterized in that the substance to be assayed is an antibody binding to a bloodgroup antigen.

15. A method according to claim 1, characterized in that a cross test is performed in that erythrocytes of unknown bloodgroup antigen composition and antibodies of unknown bloodgroup specificity are used.

16. Test kit suitable for use in a method according to any one of the preceding claims for assaying a bloodgroup antigen present on erythrocytes or an antibody binding to such a bloodgroup antigen, comprising
(i) a reagent which contains an antibody capable of binding to a bloodgroup antigen or contains a bloodgroup antigen present on erythrocytes, and
(ii) a solid phase which is a porous membrane permeable to erythrocytes, on which an immunoglobulin-binding material is immobilized.

## Patentansprüche

1. Verfahren zur Bestimmung eines Analyts bestehend aus einem auf Erythrozyten vorhandenen Blutgruppenantigen, oder einem Antikörper, der an ein solches Blutgruppenantigen bindet in einer Probe, umfassend die Behandlung der Probe mit einem Reagenz, das einen Antikörper, der in der Lage ist, an ein Blutgruppenantigen zu binden oder ein auf Erythrozyten vorhandenes Blutgruppenantigen enthält, wobei ein Komplex aus einem auf Erythrozyten vorhandenen Blutgruppenantigen und einem daran gebundenen Antikörper gebildet wird, wenn das Reagens einen Antikörper enthält, der an ein in der Probe vorhandenes Blutgruppenantigen binden kann oder die Probe enthält einen Antikörper, der an ein im Reagens vorhandenes Blutgruppenantigen bindet; weiterhin umfassend die Trennung dieses Komplexes, wenn einer gebildet wurde, von nicht komplexierten Erythrozyten und der Nachweis davon, dadurch **gekennzeichnet**, daß die Erythrozyt-enthaltenden Komplexe, wenn welche geformt werden, von nicht komplexierten Erythrozyten durch Filtration mit einer festen Phase, die eine poröse, für nicht komplexierte Erythrozyten durchlässige Membran ist, getrennt wird, wobei auf der Membran ein Immunglobulin bindendes Material immobilisiert ist.

2. Verfahren gemäß Anspruch 1, dadurch **gekennzeichnet**, daß die Probe aus Blut, Blutplasma, Blutserum, einer Blutfraktion oder einem Überstand eines Hybridoms besteht.

3. Verfahren gemäß Anspruch 1, dadurch **gekennzeichnet**, daß eine Membran aus Zellulose-Esterpolymer, Nitrozellulose, (modifiziertem) Polyvinylidenfluorid (PVDF), (modifiziertem) Polytetrafluorethylen (PTFE), (modifiziertem) Polyethersulfon, Glasfaser, (modifiziertem) Polycarbonat oder (modifiziertem) Polyamid (Nylon welches modifiziert oder nicht modifiziert sein kann) verwendet wird.

4. Verfahren gemäß Anspruch 1, dadurch **gekennzeichnet**, daß die poröse Membran Poren von wenigstens rund 2 µm, bevorzugt von wenigstens rund 3 µm hat.

5. Verfahren gemäß Anspruch 1, dadurch **gekennzeichnet**, daß in Kombination mit der porösen Membran ein hydrophober Filter verwendet wird.

6. Verfahren gemäß Anspruch 1, dadurch **gekennzeichnet**, daß als Immunglobulin bindendes Material ein Anti-Ig, Anti-IgG, Anti-IgA oder Anti-IgM verwendet wird.

7. Verfahren gemäß Anspruch 1, dadurch **gekennzeichnet**, daß als Immunglobulin bindendes Material ein Immunglobulin bindendes bakterielles Protein verwendet wird.

8. Verfahren gemäß Anspruch 7, dadurch **gekennzeichnet**, daß als Immunglobulin bindendes bakterielles Protein Protein A, Protein G, Protein L oder Protein H verwendet wird.

9. Verfahren gemäß Anspruch 1, dadurch **gekennzeichnet**, daß als Immunglobulin bindendes Material ein rekombinantes Protein, welches Immunglobulin bindende Eigenschaften hat, wie Protein L/G oder Protein A/G, verwendet wird.

10. Verfahren gemäß Anspruch 1, dadurch **gekennzeichnet**, daß die Filtration durch Zentrifugation gefördert wird.

11. Verfahren gemäß Anspruch 1, dadurch **gekennzeichnet**, daß der Filtration eine Zentrifugation vorangeht, bei der die Erythrozyten, die komplexiert oder nicht komplexiert sein können, von der Probe abgetrennt werden.

12. Verfahren gemäß Anspruch 1, dadurch **gekennzeichnet**, daß die Filtration durch eine Zentrifugation gefördert wird, während gleichzeitig die komplexierten oder nicht komplexierten Erythrozyten von der Probe abgetrennt werden.

13. Verfahren gemäß Anspruch 1, dadurch **gekennzeichnet**, daß die zu bestimmende Substanz ein auf Erythrozyten vorhandenes Blutgruppenantigen ist.

14. Verfahren gemäß Anspruch 1, dadurch **gekennzeichnet**, daß die zu bestimmende Substanz ein Antikörper ist, der an ein Blutgruppenantigen bindet.

15. Verfahren gemäß Anspruch 1, dadurch **gekennzeichnet**, daß ein Kreuztest durchgeführt wird, wobei Erythrozyten mit einer unbekannten Blutgruppenantigen-Zusammensetzung und Antikörper mit einer unbekannten Blutgruppen-Spezifität verwendet werden.

16. Testkit, das geeignet ist, in einem Verfahren gemäß einem der vorherigen Ansprüche zur Bestimmung eines auf Erythrozyten vorhandenen Blutgruppenantigens oder eines Antikörpers, der an ein solches Blutgruppenantigen bindet, verwendet zu werden, umfassend
(i) ein Reagens, welches einen Antikörper, der in der Lage ist, an ein Blutgruppenantigen zu binden oder ein auf Erythrozyten vorhandenes Blutgruppenantigen enthält, und
(ii) eine feste Phase, die eine für Erythrozyten durchlässige poröse Membran ist, auf der Immunglobulin bindendes Material immobilisiert ist.

## Revendications

1. Procédé pour tester dans un échantillon un analyte constitué d'un antigène de groupe sanguin présent sur des érythrocytes ou un anticorps se liant à un tel antigène de groupe sanguin, comprenant le traitement de l'échantillon avec un réactif contenant un anticorps capable de se lier à un antigène de groupe sanguin ou contenant un antigène de groupe sanguin présent sur des érythrocytes, grâce à quoi un complexe de l'antigène de groupe sanguin présent sur les érythrocytes et l'anticorps lié à celui-ci est formé si le réactif contient un anticorps se liant à un antigène de groupe sanguin présent dans l'échantillon ou l'échantillon contient un anticorps se liant à un antigène de groupe sanguin présent dans le réactif, comprenant de plus la séparation de ce complexe, si l'un est formé, à partir des érythrocytes non complexés, et la détection du résultat,
caractérisé en ce que les complexes contenant les érythrocytes, s'ils sont formés, sont séparés des érythrocytes non complexés par filtration à travers une phase solide qui est une membrane poreuse perméable aux érythrocytes non complexés, sur laquelle membrane une matière liant l'immunoglobuline est immobilisée.

2. Procédé selon la revendication 1,
caractérisé en ce que l'échantillon est constitué de sang, plasma sanguin, sérum sanguin, une fraction sanguine ou une phase surnageante d'un hybridome.

3. Procédé selon la revendication 1,
caractérisé en ce qu'une membrane de polymère de cellulose-ester, de nitrocellulose, de fluorure de polyvinylidène (PVDF) (modifié), de polytétrafluoroéthylène (PTFE) (modifié), de sulfone de polyéther (modifiée), de fibre de verre, de polycarbonate (modifié), ou de polyamide (modifié) (Nylon qui peut ou peut ne pas être modifié), est utilisée.

4. Procédé selon la revendication 1,
caractérisé en ce que la membrane poreuse présente des pores d'au moins environ 2 µm, de préférence au moins environ 3 µm.

5. Procédé selon la revendication 1,
caractérisé en ce qu'un filtre hydrophobe est utilisé en combinaison avec la membrane poreuse.

6. Procédé selon la revendication 1,
caractérisé en ce que, comme matière liant l'immunoglobuline, on utilise anti-Ig, anti-IgG, anti-IgA ou anti-IgM.

7. Procédé selon la revendication 1,
caractérisé en ce que, comme matière liant l'immunoglobuline, on utilise une protéine bactérienne liant l'immunoglobuline.

8. Procédé selon la revendication 7,
caractérisé en ce que, comme protéine bactérienne liant l'immunoglobuline, on utilise la protéine A, la protéine G, la protéine L ou la protéine H.

9. Procédé selon la revendication 1,
caractérisé en ce que, comme matière liant l'immunoglobuline, on utilise une protéine recombinante ayant des propriétés de liaison à l'immunoglobuline, telle que la protéine L/G ou la protéine A/G.

10. Procédé selon la revendication 1,
caractérisé en ce que la filtration est favorisée par centrifugation.

11. Procédé selon la revendication 1,
caractérisé en ce que la filtration est précédée par une centrifugation dans laquelle les érythrocytes qui peuvent ou peuvent ne pas être complexés sont séparés de l'échantillon.

12. Procédé selon la revendication 1,
caractérisé en ce que la filtration est favorisée par centrifugation, tandis que, simultanément, les érythrocytes qui peuvent ou peuvent ne pas être complexés sont séparés de l'échantillon.

13. Procédé selon la revendication 1,
caractérisé en ce que la substance à tester est un antigène de groupe sanguin présent sur des érythrocytes.

14. Procédé selon la revendication 1,
caractérisé en ce que la substance à tester est un anticorps se liant à un antigène de groupe sanguin.

15. Procédé selon la revendication 1,
caractérisé en ce qu'un test croisé est réalisé en ce que des érythrocytes d'une composition d'antigène de groupe sanguin inconnue et des anticorps de spécificité de groupe sanguin inconnue sont utilisés.

16. Kit de test approprié pour être utilisé dans un procédé selon l'une quelconque des revendications précédentes pour tester un antigène de groupe sanguin présent sur des érythrocytes ou un anticorps se liant à un tel antigène de groupe sanguin, comprenant :
(i) un réactif qui contient un anticorps capable de se lier à un antigène de groupe sanguin ou contient un antigène de groupe sanguin présent sur des érythrocytes, et
(ii) une phase solide qui est une membrane poreuse perméable aux érythrocytes, sur laquelle une matière liant l'immunoglobuline est immobilisée.
